(19) Europäisches Patentamt European Patent Office Office européen des brevets

(11) **EP 2 687 158 B1**

(12) **EUROPEAN PATENT SPECIFICATION**

(45) Date of publication and mention of the grant of the patent:
**13.06.2018 Bulletin 2018/24**

(51) Int Cl.:
**A61B 5/107** *(2006.01)*     **A61B 5/053** *(2006.01)*

(21) Application number: **13159312.1**

(22) Date of filing: **15.03.2013**

(54) **Biometric apparatus and computer-readable storage medium storing body image creating program**

Biometrische Vorrichtung und computerlesbares Speichermedium zum Speichern eines Körperbilderzeugungsprogramm

Appareil biométrique et support de stockage lisible par ordinateur stockant un programme de création d'image de corps

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.07.2012 JP 2012160147**

(43) Date of publication of application:
**22.01.2014 Bulletin 2014/04**

(73) Proprietor: **Tanita Corporation Tokyo 174-8630 (JP)**

(72) Inventor: **Uchiyama, Tomoka Itabashi-ku, Tokyo 174-8630 (JP)**

(74) Representative: **Grünecker Patent- und Rechtsanwälte PartG mbB Leopoldstraße 4 80802 München (DE)**

(56) References cited:
**EP-A1- 1 621 131      JP-A- 2009 039 289
US-A1- 2005 059 903      US-A1- 2007 135 737
US-A1- 2009 099 457      US-A1- 2011 137 198
US-A1- 2011 295 144**

**Description**

BACKGROUND OF THE INVENTION

1. Field of the Invention

The present invention relates to a biometric apparatus and a computer-readable storage medium storing a body image creating program capable of creating a body image of a user.

2. Description of the Related art

Body composition meters of the related art include a type configured to be capable of calculating the fat percentage, the fat mass, the lean body mass, the muscle mass, the visceral fat mass, the visceral fat area, the subcutaneous fat mass of the entire body in addition to measuring the body weight, and furthermore, capable of calculating such the biometric information of the body on the part-to-part basis. Examples of such body composition meters include a body composition meter as disclosed in JP-A-2005-261488 configured to calculate biometric information of the body of a user on the part-to-part basis and then display the biometric information of the respective parts in sequence while illuminating the part corresponding to the respective items of biometric information by color coding on a human body icon display.

However, the body composition meter of the related art only displays the results of measurement of the respective parts in sequence. Therefore, in order to figure out the state of the entire body in a comprehensive manner on the basis of the results of measurement of the respective parts, the user by himself or herself needs to figure out the state by combining the results of measurement of the respective parts. Since the user may not have sufficient knowledge required for the synthetic understanding, it may be difficult to understand or may not be capable of understanding in such a case.

The body composition meter of the related art displays the results of measurement in sequence on the part-to-part basis by numerical values. The body composition meter disclosed in JP-A-2005-261488 proposes indicating which part of the body the result of measurement being displayed in numerical values corresponds by color-coded illumination of the human body icon. However, even though the result is displayed by the numerical value or the corresponding part of the body is indicated, the user can hardly know how the figure of himself or herself is or how the ratio between the fat mass and the lean body mass is through instinct, and can hardly understand the state of the entire body synthetically on the basis of the results of measurement of the respective parts. Therefore, there are users who cannot perform the figure control using the body composition meter. Document US 2011/0295144 A1 is directed at an abdominal fat measuring device. Vertical and horizontal widths of the abdomen of a subject are measured and an ellipse defined by such widths is defined as an outer shape ellipse.

SUMMARY

The invention provides a biometric apparatus according to claim 1. Accordingly, it is an object of the invention to provide a biometric apparatus and a computer-readable storage medium storing a body image creating program capable of displaying a body image which helps a user to figure out through instinct on the basis of biometric information on the entire body and the respective body parts.

In view of such circumstances, according to a first aspect of the invention, there is provided a biometric apparatus including: a biometric information acquiring unit configured to acquire biometric information of a user; a storing unit configured to store a correspondence relation between the biometric information and body part sizes; an arithmetic operation unit configured to calculate the body part sizes of the user on the basis of the biometric information acquired by the biometric information acquiring unit and the correspondence relation stored in the storing unit; an image creating unit configured to create a body image of the user on the basis of the body part sizes calculated by the arithmetic operation unit; and a display unit configured to display the body image created by the image creating unit.

Preferably, the biometric information acquired by the biometric information acquiring unit includes height, sex, and age of the user, and the correspondence relation stored in the storing unit has a ratio of the body part sizes with respect to the height, and is set according to the sex and age.

Preferably, the biometric information acquiring unit acquires body composition information as the biometric information on the entire body, and on the part to part basis by measuring the user.

Preferably, the image creating unit creates a body image indicating part or the entire figure of the user's body.

Preferably, the image creating unit creates a body image differentiating fat and lean body of the user's body part.

Preferably, the arithmetic operation unit calculates the diameters of arms and/or legs and the diameters of lean bodies of arms and/or legs as the body part sizes and the image creating unit creates a body image in which fat and lean body of the arms and/or legs are differentiated on the basis of the body part size.

Preferably, the image creating unit creates the body image by correcting a model data relating to a figure stored in the storing unit on the basis of the body part sizes calculated by the arithmetic operation unit.

Preferably, the arithmetic operation unit calculates the abdominal circumference, the length of the body trunk, the lengths of the arms, the lengths of the legs, fat volumes and lean body volumes of the respective body parts, the average cross-sectional area of the fat and the average cross-sectional area of the lean body of the respective body parts, the arm diameters, the leg diameters, the acromial width, the infra-axillary width, the navel height, the waist height, and the long axis of abdominal circumference as the body part sizes, and the image creating unit creates an body image of the front of the user on the basis of the body part sizes.

Preferably, the arithmetic operation unit calculates the infra-axillary thickness, the short axis of abdominal circumference, and the length from the back to the centers of the arms as body part sizes and the image creating unit creates a body image of the side surface of the user on the basis of the body part sizes.

Preferably, the image creating unit creates a body image imitating the cross section of the body parts of the user, which is a body image differentiating fat and lean body.

Preferably, the image creating unit creates a body image imitating an abdominal cross-section of the user including an ellipsoidal model of an abdominal circumference cross-section of the user and an ellipsoidal model of the visceral fat area of the user.

According to the second aspect of the invention, there is provided a computer-readable storage medium storing a body image creating program executed by an information processing apparatus capable of receiving biometric information of a user for creating a body image of the user on the basis of the biometric information, wherein the program causes the information processing apparatus to execute; a size calculating process for calculating body part sizes of the user on the basis of a correspondence relation between the biometric information and the body part sizes; and an image creating process for creating a body image of the user on the basis of the body part sizes calculated by the size calculating process.

Preferably, the information processing apparatus is a computer or a mobile computer, and a computer-readable storage medium storing the body image creating program is configured to cause a display unit of the information processing apparatus to display the body image created by the image creating process.

According to the biometric apparatus and a computer-readable storage medium storing the body image creating program of the invention, the user is capable of figuring out the state of the body through instinct by the creation and the display of the body image of the user, which allows the user to synthetically understand diet or the like.

In general, it may be said that the motivation for starting diet is a moment when one sees himself or herself in photos or in a mirror. By confirming the body image using the biometric apparatus and a computer-readable storage medium storing the body image creating program of the invention on the daily basis, the user is capable of understanding his or her figure quickly and easily. Accordingly, the user may have a consciousness relating to his or her own figure further in detail.

BRIEF DESCRIPTION OF THE PREFERRED EMBODIMENT

FIG. 1 is a perspective view of a biometric apparatus according to a first embodiment of the invention;
FIG. 2 is a block diagram of the biometric apparatus illustrated in FIG. 1;
FIG. 3 is a flowchart illustrating a flow of a process of measuring and displaying biometric information using the biometric apparatus of the first embodiment;
FIG. 4 is a flowchart illustrating a flow of a size calculating process and an image creating process of the biometric apparatus;
FIG. 5 is a table of list of ratios of the body part sizes with respect to heights set according to the sexes and the ages.
FIG. 6A is a front perspective view for explaining the respective body parts listed in FIG. 5;
FIG. 6B is a side view for explaining the respective body parts listed in FIG. 5 viewed from the X direction;
FIG. 7 is a cross-sectional view for explaining abdominal circumference sizes viewed from the Z direction;
FIG. 8A is a cross-sectional view for explaining the fat and the lean body of an arm and a leg taken along the Z direction;
FIG. 8B is a cross-sectional view for explaining the fat and the lean body of the arm and the leg taken along the direction orthogonal to the Z direction;
FIG. 9A is a schematic front view for explaining an example of creating a body image in a dot matrix pattern on the basis of the respective calculated body part sizes viewed from the Y direction;
FIG. 9B is a side view for explaining the example of creating the body image in a dot matrix pattern on the basis of the respective calculated body part sizes viewed from the X direction;
FIG. 10A illustrates an example of body image displayed in a case of a person having a standard figure;
FIG. 10B illustrates an example of body image displayed in a case of a person having a fatty figure in comparison with the case of FIG. 10A;

FIG. 11 illustrates an example of a basic model shape used for determining the shapes of body trunk, arms, and legs;

FIG. 12 illustrates an example of a body image imitating an abdominal cross-section of a user taken along the direction orthogonal to the Z direction;

FIG. 13A illustrates an example of the body image imitating the abdominal cross-section of the user having less subcutaneous fat and visceral fat than standard; and

FIG. 13B illustrates an example of the body image imitating the abdominal cross-section of the user having larger amount of subcutaneous fat and visceral fat than standard.

## DESCRIPTION OF PREFERRED EMBODIMENT

(First Embodiment)

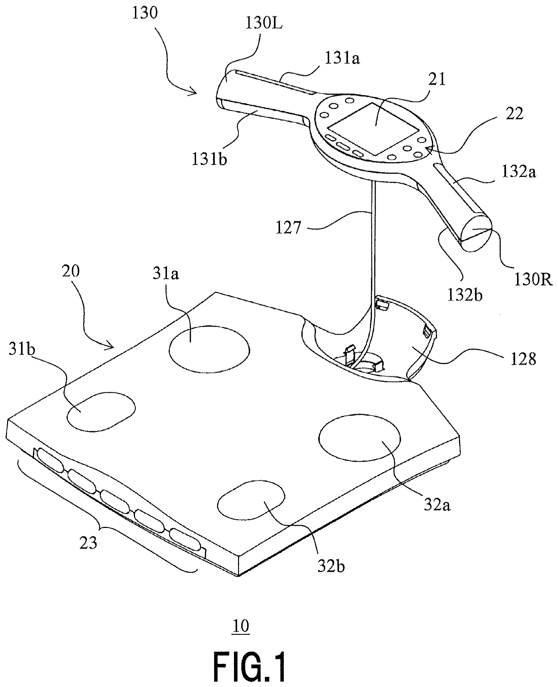

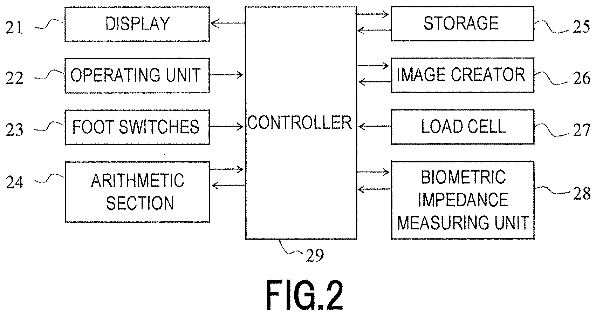

Referring now to the drawings, a biometric apparatus according to a first embodiment of the invention will be described in detail. In the first embodiment, an example in which the invention is applied to a weighting machine (a weighting machine with body composition meter) capable of measuring the body composition will be described. FIG. 1 is a perspective view showing a configuration of the biometric apparatus according to the first embodiment of the invention, and FIG. 2 is a block diagram of the biometric apparatus in FIG. 1.

As illustrated in FIG. 1 and FIG. 2, a weighing machine 10 as a biometric apparatus includes a body 20 and a handle unit 130 electrically connected to the body 20 by a connecting line 127 and demountably mounted to a housing 128, and further includes grip portions 130L and 130R, a display 21, an operating unit 22, foot switches 23, an arithmetic section 24, a storage 25, an image creator 26, a load cell 27 as a weight measuring portion, a biometric impedance measuring unit 28, electrodes 31a and 32a for power distribution and electrodes 31b and 32b for measurement capable of coming into contact with the feet of the user, electrodes 131a and 132a for power distribution and electrodes 131b and 132b for measurement capable of coming into contact with the hands of the user, a controller 29, and a power source (not illustrated) that supplies an electric power. As illustrated in FIG. 1, the body 20 includes the electrodes 31a and 32a for power distribution and the electrodes 31b and 32b for measurement, and the foot switches 23 including a plurality of switches arranged thereon. The handle unit 130 includes the display 21, the operating unit 22, two grip portions 131L and 131R arranged on both sides, the electrodes 131a and 132a for power distribution and the electrodes 131b and 132b for measurement. In FIG. 2, the arithmetic section 24, the storage 25, the image creator 26, the load cell 27, the biometric impedance measuring unit 28, and the controller 29 arranged in the interior of the weighing machine 10 are illustrated. These members, being internal mechanisms, are not illustrated in FIG. 1.

Detailed configurations of the respective members will be described below.

In the invention, the biometric information includes (1) biometric information acquired by measuring the user directly by the biometric apparatus (mainly including the body weight and the biometric impedance or the like) (2) biometric information acquired by the user by operating the operating unit 22 and inputting entries (mainly including age, sex, height or the like), and (3) biometric information acquired by calculating the biometric information by applying a predetermined regression formula thereto (mainly including the fat percentage, the fat mass, the lean body mass, the muscle mass, the visceral fat mass, the visceral fat level, the visceral fat area, the subcutaneous fat mass, the amount of basal metabolism, the bone mass, the body moisture percentage, the BMI (Body Mass Index), the intercellular fluid volume, and the extracellular fluid volume or the like of the entire body and of the respective body parts).

The display 21 as a display unit displays a body image of the user created by the image creator 26, the biometric information measured by the load cell 27 or the biometric impedance measuring unit 28, the biometric information input by the user by operating the operating unit 22 or the foot switches 23 or other items of information. As the display 21, a unit using liquid crystal such as a full-dot LCD (Liquid Crystal Display) is employed.

The operating unit 22 is an input unit configured to input the biometric information such as height, sex, and age, and preset entries for individuals. The input personal biometric information or the preset entries are stored in the storage 25, and are displayed on the display 21. The types of the operating unit 22 which may be employed include, for example, a button-type, a touch sensor type, and a dial type. In the invention, the operating unit 22 functions also as a biometric information acquiring unit.

The foot switches 23 are connected to the controller 29, and are configured to turn on and off the weighing machine 10 by sending signals to the controller 29 and, if the personal biometric information or the preset entries are stored in the storage 25 in advance, call up the the personal biometric information or the preset entries. For example, when a plurality of users use the weighing machine 10, the respective foot switches 23 may be allocated to the respective users, so that the users may call up their own biometric information or the preset entries by pushing the allocated foot switches 23 . Here, the term "preset entries" means entries set by the user for using the weighing machine 10. For example, the size of characters or signs of the biometric information displayed on the display 21 or types of the biometric information are included.

The arithmetic section 24 as an arithmetic operation unit executes various arithmetic operation processes such as applying information such as height, age, and sex input by the user, or biometric information such as measured weight and biometric impedance of the user to a predetermined regression formula and calculates the biometric information such as the fat percentage under the control of the controller 29. In particular, in the invention, the arithmetic section 24 calculates body part sizes of the user on the basis of the biometric information and a correspondence relation (described later) acquired by the biometric information acquiring unit (the operating unit 22, the load cell 27, the biometric impedance measuring unit 28). The results of calculation are stored in the storage 25. In the invention, the arithmetic section 24 functions also as the biometric information acquiring unit.

The controller 29 may be provided with above-described functions of the arithmetic section 24.

The storage 25 as a storing unit is the storing unit configured with a ROM (non-volatile memory (Read Only Memory)), a RAM (volatile memory (Random Access Memory)) and stores various data. In particular, in the invention, the correspondence relation between the biometric information and the body part sizes is stored in the storage 25. The storage 25 in this embodiment is a computer-readable storage medium storing the body image creating program according to the invention.

The image creator 26 as an image creating unit creates a body image of the user on the basis of the body part sizes of the user calculated by the arithmetic section 24. In particular, in this embodiment, the image creator 26 creates body images of the respective body parts of the user and a body image combining the images of the respective body parts.

The arithmetic section 24 and/or the controller 29 may be provided with the above-described functions of the image creator 26.

The load cell 27 as the biometric information acquiring unit includes a flexure element, which is a metallic member, deformed according to the load and a strain gauge stuck to the flexure element. When the user rides on the weighing machine 10, the flexure element of the load cell 27 is deflected by the load of the user, and hence the strain gauge is expanded and contracted, so that the value of resistance (output value) of the strain gauge varies according to the expansion and contraction thereof. The controller 29 causes the arithmetic section 24 to perform an arithmetic operation of the weight from the difference between an output value from the load cell 27 when no load is applied (zero point) and an output value when a load is applied, so that the weight of the user is measured. A configuration relating to the measurement of the weight using the load cell 27 may be the same as the configuration of general weighing machines.

The biometric impedance measuring unit 28 as the biometric information acquiring unit includes (1) the electrodes 31a and 32a for power distribution and the electrodes 31b and 32b for measurement configured to come into contact with the feet of the user, (2) the electrodes 131a and 132a for power distribution and the electrodes 131b and 132b for measurement which come into contact with the hands of the user, (3) a constant current supply unit (not illustrated) connected to the electrodes 31a and 32a for power distribution and the electrodes 131a and 132a for power distribution and configured to supply a high frequency weak constant current, and (4) a voltage measuring unit (not illustrated) connected to the electrodes 31b and 32b for measurement and the electrodes 131b and 132b for measurement and configured to measure the potential difference of the living body.

As illustrated in FIG. 1, the electrodes 31a and 32a for power distribution and the electrodes 31b and 32b for measurement are arranged apart from each other on the upper surface of the body 20 of the weighing machine 10, and configured as electrodes for the legs which come into contact with the bottoms of the left and right feet when the user gets on the weighing machine 10. The electrodes 131a and 132a for power distribution and the electrodes 131b and 132b for measurement are arranged on the peripheral surfaces of the grip portions 130L and 130R of the handle unit 130 of the weighing machine 10 so as to be apart from each other, and are configured as electrodes for the hands which come into contact with palms of the left and right hands when the user grips the grip portions 130L and 130R.

The measurement of the biometric impedance of the entire body and the respective body parts of the user is performed, for example, as follows.

(1) the measurement of the biometric impedance of the entire body is performed by supplying an electric current using the electrode 131a for power distribution and the electrode 31a for power distribution and measures the potential difference in a current route flowing from the left hand, the left arm, the breast, the abdominal portion, the left leg and the left foot between the electrode 131b for measurement in contact with the left hand, and the electrode 31b for measurement in contact with the left foot.

(2) the measurement of the biometric impedance of the right leg is performed by supplying an electric current using the electrode 132a for power distribution and the electrode 32a for power distribution and measures the potential difference in a current route flowing from the right hand, the right arm, the breast, the abdominal portion, the right leg and the right foot between the electrode 31b for measurement in contact with the left foot, and the electrode 32b for measurement in contact with the right foot.

(3) the measurement of the biometric impedance of the left leg is performed by supplying an electric current using the electrode 131a for power distribution and the electrode 31a for power distribution and measures the potential difference in a current route flowing from the left hand, the left arm, the breast, the abdominal portion, the left leg and the left foot between the electrode 31b for measurement in contact with the left foot, and the electrode 32b for measurement in contact with the right foot.

(4) the measurement of the biometric impedance of the right arm is performed by supplying an electric current using the electrode 132a for power distribution and the electrode 32a for power distribution and measures the potential difference in a current route flowing from the right hand, the right arm, the breast, the abdominal portion, the right leg and the right foot between the electrode 131b for measurement in contact with the left hand, and the electrode 132b for measurement in contact with the right hand.

(5) the measurement of the biometric impedance of the left arm is performed by supplying an electric current using the electrode 131a for power distribution and the electrode 31a for power distribution and measures the potential difference in a current route flowing from the left hand, the left arm, the breast, the abdominal portion, the left leg and the left foot between the electrode 131b for measurement in contact with the left hand, and the electrode 132b for measurement in contact with the right hand.

In this manner, the weighing machine 10 flows an electric current from the respective electrodes for power distribution to predetermined parts of the body of the user, so that the potential difference generated in the corresponding current route may be measured. The controller 29 is configured to be capable of causing the arithmetic section 24 to calculate the biometric impedance of the user on the basis of the electric current and the respective values of the potential difference as described above. In addition, the controller 29 is configured to be capable of causing the arithmetic section 24 to calculate the fat percentage or the visceral fat level and so on by applying the biometric impedance calculated or measured in this manner or the biometric information such as weight, age, sex, height to the predetermined regression formula. A configuration relating to the measurement of the biometric impedance may be the same as the configuration of general body fat scales or the body composition meters.

As illustrated in FIG. 2, the display 21, the operating unit 22, the foot switches 23, the arithmetic section 24, the storage 25, the image creator 26, the load cell 27, and the biometric impedance measuring unit 28 is electrically connected to the controller 29, and the controller 29 controls the operations of these units. Furthermore, a power source (not illustrated) is connected to the controller 29. As the power source, batteries for supplying an electric power to operate the weighing machine 10 or an external power source may be used.

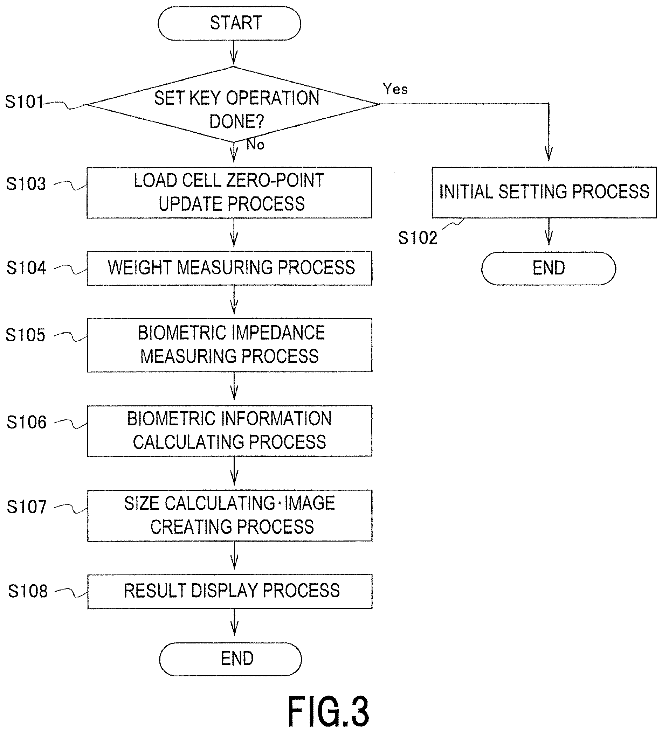

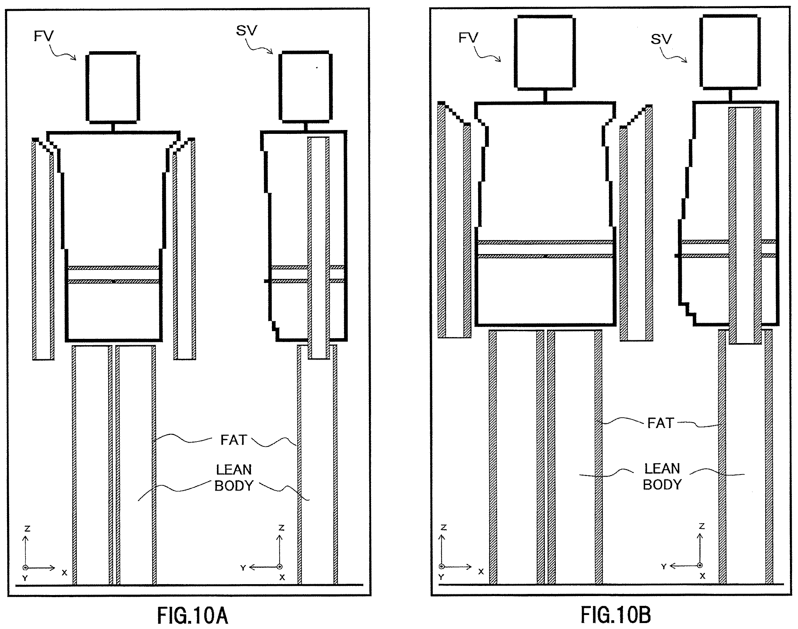

Subsequently, referring to FIG. 3 to FIGs. 10A and 10B, a process from the measurement of the biometric information to the creation and display of the body image of the user in the weighing machine 10 will be described.

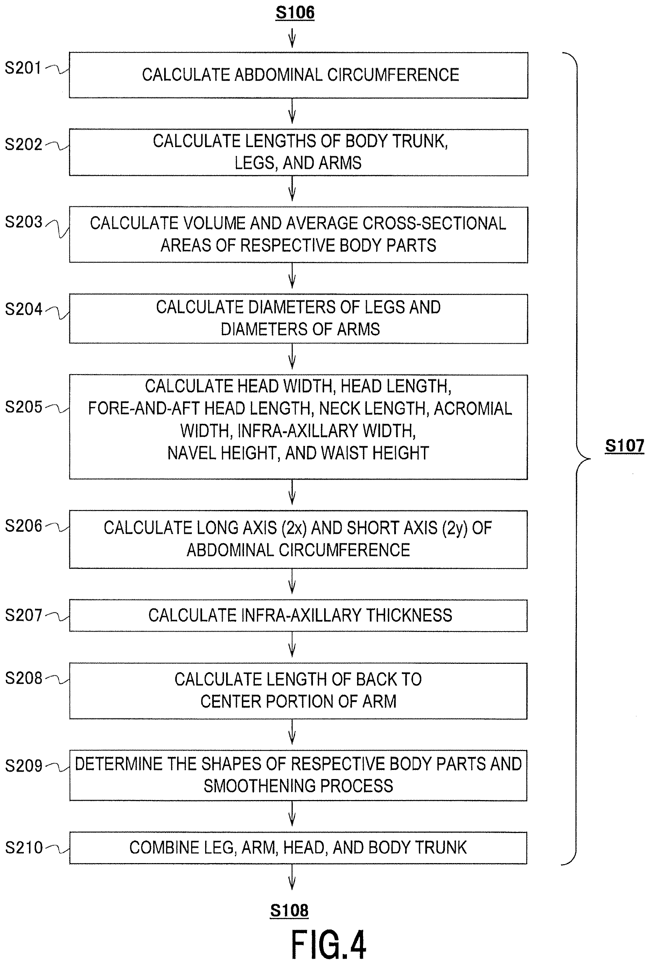

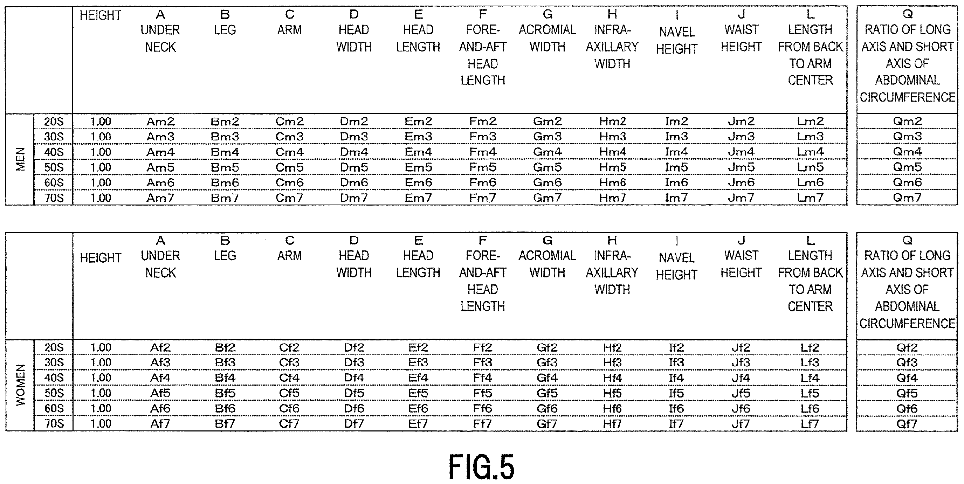

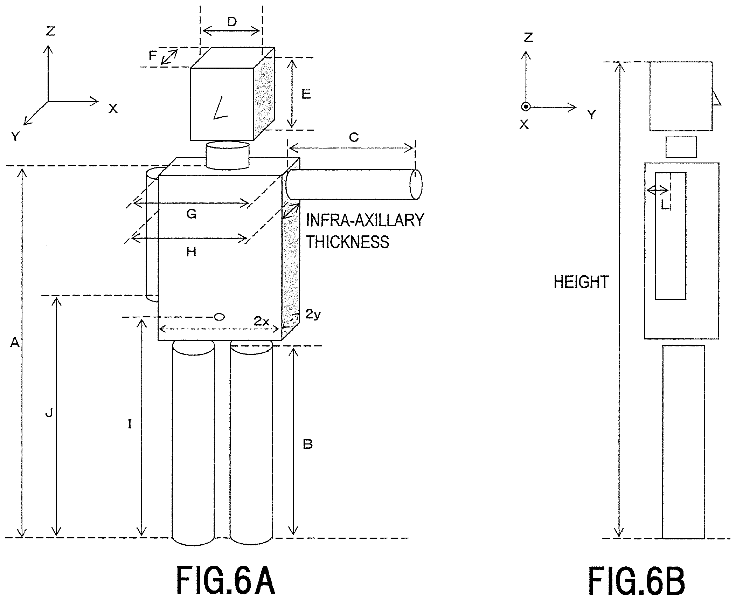

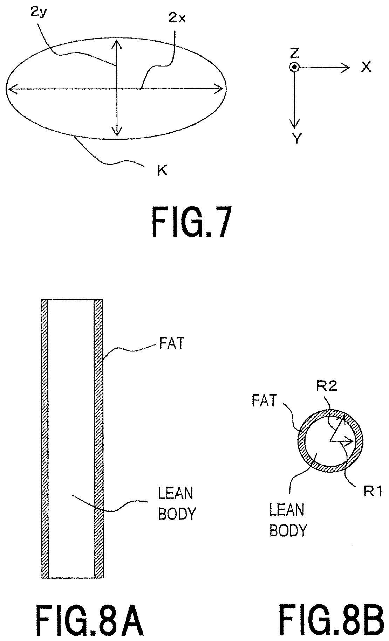

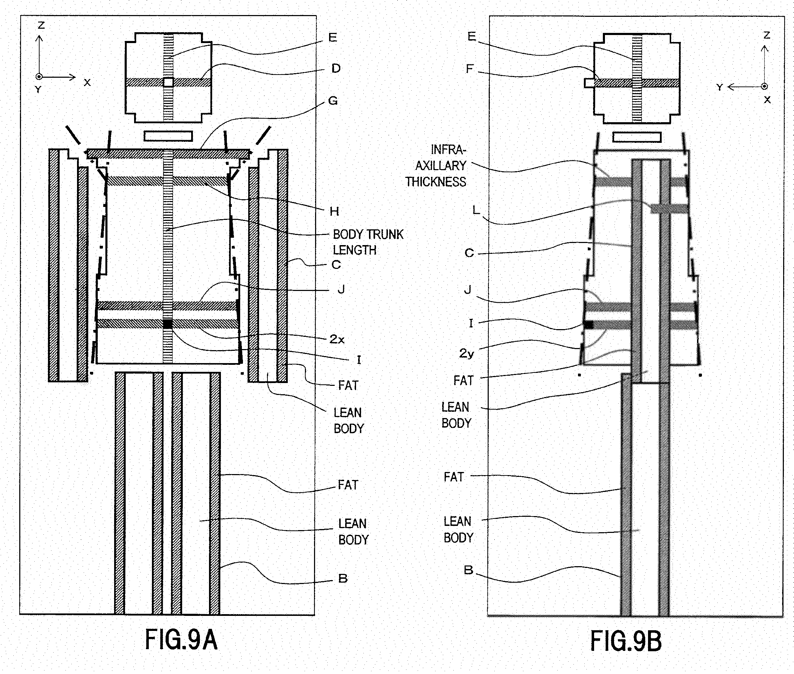

FIG. 3 is a flowchart illustrating a flow of a process of measuring and displaying biometric information using the biometric apparatus of the first embodiment, FIG. 4 is a flowchart illustrating a flow of a size calculating process and an image creating process of the biometric apparatus, FIG. 5 is a table of list of ratios of the body part sizes with respect to heights set according to the sexes and the ages, FIG. 6A is a front perspective view for explaining the respective body parts listed in FIG. 5; FIG. 6B is a side view for explaining the respective body parts listed in FIG. 5 viewed from the X direction, FIG. 7 is a cross-sectional view for explaining abdominal circumference sizes viewed from the Z direction, FIG. 8A is a cross-sectional view for explaining the fat and the lean body of the arm and the leg taken along the Z direction, FIG. 8B is a cross-sectional view for explaining the fat and the lean body of the arm and the leg taken along the direction orthogonal to the Z direction, FIG. 9A is a schematic front view for explaining an example of creating a body image in a dot matrix pattern on the basis of the respective calculated body part sizes viewed from the Y direction, FIG. 9B is a side view for explaining the example of creating the body image in a dot matrix pattern on the basis of the respective calculated body part sizes viewed from the X direction, FIG. 10A illustrates an example of body image displayed in a case of a person having a standard figure, and FIG. 10B illustrates an example of body image displayed in a case of a person having a fatty figure in comparison with the case of FIG. 10A.

When the operation of the weighing machine 10 in the state not in use is started, the controller 29 determines whether the weighing machine 10 is started or not by the operation of a set key for activating a setting mode from among the operating unit 22 or the foot switches 23 (Step S101).

When the operation of the set key is performed (Yes in Step S101), the controller 29 displays a predetermined setting screen on the display 21, and performs an initial setting process (setting mode) (Step S102) . In the initial setting process, the controller 29 performs the setting process by causing the storage 25 to store items such as height, sex, age, and input by the user through the operation of the operating unit 22 or the foot switches 23 while viewing the setting screen, and then terminates the operation of the weighing machine 10 by converting the weighing machine 10 to the state not in use.

In contrast, when the start of the operation of the weighing machine 10 is not caused by the operation of the set key, but, for example, by the operation of the start key, the controller 29 performs a measuring mode (No in Step S101) . The controller 29 performs a zero-point update process that activates the load cell 27 and acquires the output value at the time of no load, and set the corresponding point as the zero point (Step S103) .

Subsequently, the controller 29 performs a weight measuring process (Step S104). More specifically, the controller 29 displays predetermined guidance messages on the display 21 for the user to give the user an instruction to get on the body 20 of the weighing machine 10, acquires an output value of the load cell 27 when the user gets on the body 20, and causes the arithmetic section 24 to perform an arithmetic operation of the weight value. The controller 29 stores the result of measurement of the weight in the storage 25.

Subsequently, the controller 29 starts a biometric impedance measuring process (Step S105). More specifically, the controller 29 causes the display 21 to display the predetermined guidance message for the user, activates the biometric impedance measuring unit 28 to acquire the output value of the biometric impedance measuring unit 28 (the potential difference measured in the predetermined current route passing in the body of the user), and causes the arithmetic section 24 to perform the arithmetic operation of the biometric impedance value. The controller 29 stores the result of measurement of the biometric impedance in the storage 25.

The controller 29 causes the arithmetic section 24 to calculate other biometric information (the body composition information) on the basis of the results of measurement in the weight measuring process (Step S104) and the biometric impedance measuring process (Step S105) described above (Step S106). In other words, the arithmetic section 24, the load cell 27, the biometric impedance measuring unit 28, and the controller 29 as the biometric information acquiring unit are configured to acquire the body composition information as the biometric information of the entire part of the body and on the part-to-part basis by measuring the user. In this embodiment, the body composition information as the biometric information to be calculated includes the fat percentage, the fat mass, the lean body mass, the muscle mass, the visceral fat mass, the visceral fat area, and the subcutaneous fat mass of the entire body or of the respective body parts. The arithmetic operation reads the results of measurement of the weight measuring process and the biometric impedance measuring process from the storage 25, and is performed according to the program stored in the storage 25 in advance.

The controller 29 causes the arithmetic section 24 or the image creator 26 to perform the size calculating process and the image creating process (FIG. 4) described later in detail (Step S107) .

The controller 29 causes the display 21 to display the result of measurement of the biometric information or the created body image (see FIGs. 10A and 10B) (Step S108), and then the controller 29 converts the weighing machine 10 to the state not in use and terminates the operation of the weighing machine 10.

Subsequently, the size calculating process and the image creating process (FIG. 4) will be described. The size calculating process and the image creating process are processes for displaying a body image reflecting the current biometric information of the user on the display 21. In particular, in this embodiment, in order to display the body image on the display 21 in a dot matrix pattern, that is, in order to determine the sizes of the respective body parts of the body image displayed in the dot matrix pattern, a body image of the entire body which can be displayed in a dot matrix pattern is created (image creating process) by firstly calculating the sizes of the actual body parts of the user from the current biometric information of the user respectively as estimated values (size calculating process) and, secondly, creating the body images of the respective body parts at the same ratio as the actual body part sizes of the user and are combined to each other.

Here, in order to calculate the actual body part sizes of the user from the current biometric information of the user respectively as the estimated values, a database which serves to obtain the estimated values of the body part sizes from a measured value of the current biometric information may be created in advance. In this embodiment, the correspondence relation between the biometric information (specifically, sex and age) and the body part sizes as illustrated in FIG. 5 are stored in the storage 25.

In the list (correspondence relation) illustrated in FIG. 5, an example including a database of the respective ratios of a length under neck A, a leg length B, an arm length C, a head width D, a head length E, a fore-and-aft head length F, an acromial width G, an infra-axillary width H, a navel height I, a waist height J, and a length from the back to the arm center L to the height on the basis of the hypothesis that the lengths of the respective body parts are proportional to the height by sex and age group, and a database of the ratio of long axis and short axis of abdominal circumference Q by sex and age group is shown. The lengths of the respective body parts are schematically illustrated in FIGs. 6A and 6B By creating the correspondence relation as described above, for example, an estimated value of the leg length B of, for example, a male user in his 30s, being 170 cm tall can be obtained easily by an expression "1.7 (m) $\times$ Bm3". The ratio of the length of the respective body parts with respect to the height may be created on the basis of the statistical data relating to the human body dimensions.

The size calculating process and the image creating process will be described below along the flow chart in FIG. 4.

The abdominal circumference which is a length around the waist passing the navel (the abdominal circumference diameter, K in FIG. 7) is calculated (Step S201).

The estimated value of the abdominal circumference is calculated by the following regression formula (1) shown below.

$$\text{The abdominal circumference } K = a1 \times BMI + a2 \times VFM + a3 \times SFM + a4 \cdots (1)$$

Here, a1, a2, a3, a4 are coefficients and, for example, the static is taken for an unspecified number of tested subjects, and a constant which can calculate the abdominal circumference K from BMI, VFM, SFM is set as needed in advance on the basis of the statistical results. The BMI may be obtained from "weight (kg) / height (m) /height (m)". The VFM means Visceral Fat Mass, and the SFM means Subcutaneous Fat Mass.

The lengths of the body trunk, the legs, and the arms are calculated, respectively (Step S202).

Estimated values of the respective lengths of the body trunk, the legs, and the arms may be calculated on the basis of the height, sex, and age of the user by using the database illustrated in FIG. 5. The length of the body trunk is a value obtained by subtracting the leg length B from the length under neck A.

The volume and the average cross-sectional area of the each body part are calculated (Step S203).

The volume and the average cross-sectional area of the each body part are calculated as estimated values of the fat volume and the lean body volume of the each body part, and the average cross-sectional surface of fat and the average cross-sectional area of lean body of the each body part from the fat mass and the lean body mass of the each body part calculated in Step S106. Even when the weights of fat, muscle, and bones are the same, the volumes are different because the density is different. Therefore, in order to reflect the silhouette (the circumference diameter and the width) correctly on the body image, the volume and the average cross-sectional area of fat tissue and lean body tissue may be calculated respectively.

In order to calculate fat volume and lean body volume from the fat mass and the lean body mass of the each body part calculated in Step S106, the following expressions (2) and (3) may be used.

$$\text{lean body volume } M'(cm^3) = (M \times 10^3)/1.1 \cdots (2)$$

$$\text{fat volume } N'(cm^3) = (N \times 10^3)/0.9 \cdots (3)$$

Where M is a lean body mass (kg) and N is a fat mass (kg). The density of the lean body is 1.1g/cm$^3$, and the density of fat is 0.9 g/cm$^3$.

The average cross-sectional area of fat of the each body part may be calculated by dividing the fat volume calculated as described above by the length of the body part. In the same manner, the average cross-sectional area of lean body of the each body part may be calculated by dividing the lean body volume calculated as described above by the length of the body part. In this manner, by calculating the volume and the average cross-sectional area of fat tissue and lean body tissue separately and then adding the fat volume and the lean body volume of the same body part, the volume of the corresponding body part is calculated. In the same manner, by adding the fat average cross-sectional area and the lean body average cross-sectional area of the same body part, the average cross-sectional area of the corresponding body part is calculated.

The diameters of the legs and the diameters of the arms are calculated (Step S204).

In this embodiment, assuming that the legs and the arms have a column shape (see FIGs. 8A and 8B), the diameters of the legs and the diameters of the arms are calculated by being estimated from the volume and the average cross-sectional area of the legs and the volume and the average cross-sectional area of the arms calculated in Step S203.

Here, as regards the lean body surface area of the arm, the following expression (4) is satisfied.

$$\text{Lean body surface area of arm} = \text{lean body volume } M' \text{ of arm} /\text{arm length } C \cdots (4)$$

As illustrated in FIG. 8B, when a radius R1 of the lean body of the arm is used, the lean body cross-sectional area of the arm is equal to $\pi \times R1^2$. Therefore, from the expression (4), the radius R1 of the lean body of the arm is calculated by the following expression (5).

$$R1 = \sqrt{(\text{lean body volume } M' \text{ of arm}/\text{arm length } C)/\pi} \cdots (5)$$

Accordingly, the diameter of the lean body of the arms may be calculated by "R1×2". In addition, a radius R2 of the arm illustrated in FIG. 8B satisfies the relation of the following expression from the above-described expression (3).

$$R2= \sqrt{(((\text{fat volume N' of arm/arm length C})/\pi) +R1^2)}$$

Accordingly, the diameter (thickness) of the arm including the fat and the lean body mays be calculated by "R2×2".

In the same manner, the diameters of the lean body of the legs (R1×2) and the diameters of the legs including fat and the lean body (R2×2) are calculated.

The head width D, the head length E, the fore-and-aft head length F, the neck length, the acromial width G, the infra-axillary width H, the navel height I, and the waist height J are calculated (Step S205). These values may be calculated as an estimated value on the basis of the height, sex, and age of the user by using the database illustrated in FIG. 5. The neck length is calculated by "height-length under neck A-head length E".

The long axis of the abdominal circumference (2x in FIG. 7) and the short axis (2y in FIG. 7) are calculated (Step S206).

The long axis and the short axis of the abdominal circumference is calculated as an estimated value on the basis of the abdominal circumference K calculated by Step S201 and a database of age, sex, and ratio of long axis and short axis of abdominal circumference Q of the user. The ratio of long axis and short axis of abdominal circumference Q is the ratio between the length of the long axis (2x in FIG. 7) and the length of the short axis (2y in FIG. 7) when the abdominal circumference cross sections assumed to be an ellipsoidal shape.

The long axis (2x) and the short axis (2y) of the abdominal circumference may be obtained from a half long axis x and a half short axis y of the abdominal circumference, and the estimation expressions of the half long axis x and the half short axis y are the following expressions (6) and (7).

$$x= (\beta-\sqrt{(\beta^2 -4\alpha\cdot K)})/2\alpha \;\cdot\cdot\cdot\; (6)$$

$$y=Q\cdot x \;\cdot\cdot\cdot\; (7)$$

Here,

$$\alpha= (1-Q)^2/2.2$$

$$\beta= \pi\sqrt{(2(1+Q^2))}$$

where Q is the ratio of long axis and short axis of abdominal circumference, and K is an abdominal circumference.

The ellipsoidal shape of the abdominal circumference may be changed in ellipsoidal ratio between a visceral fat pyknic type and a subcutaneous fat pyknic type. For example, the shape of the abdominal circumference of the visceral fat pyknic type is closer to a true circle in comparison with the subcutaneous fat pyknic type, and consequently, in the body image of the body trunk described later, the abdominal portion develops to the front.

The ratio of long axis and short axis of abdominal circumference Q may be determined (corrected) considering the age factor using the ratio between the visceral fat area and the surface area of subcutaneous fat.

The infra-axillary thickness (see FIG. 6A) is calculated (Step S207).

The infra-axillary thickness is calculated by estimating from the infra-axillary width H calculated in Step S205 and the ratio of long axis and short axis of abdominal circumference Q.

The estimation expression of the infra-axillary thickness is the following expression (8).

Infra-axillary thickness = infra-axillary width H × ratio

of long axis and short axis of abdominal circumference Q ·

· (8)

The length L from the back to the center portion of the arm is calculated (Step S208).

The length L from the back to the center portion of the arm may be calculated as an estimated value on the basis of the height, sex, and age of the user by using the database illustrated in FIG. 5.

In Steps from Step S201 to Step S208, the size calculating process for calculating the actual body part sizes of the user from the current biometric information of the user respectively as estimated values is terminated. Subsequently, an image creating process configured to create the entire body image by creating the body images of the respective body parts by reducing the actual respective body part sizes of the user calculated as described above to the sizes which allow the dot-matrix display on the display 21 and combining the body images (Step S209 to Step S210) will be described.

The image creator 26 determines the shape of the respective body parts and performs a smoothing process (Step S209).

In this embodiment, a simple model in which the legs and the arms have a column shape, and the shape of the body trunk is an elliptical cone shape (or the truncated elliptical cone) from among the body parts will be assumed in this embodiment. However, the invention is not limited thereto, and an arbitrary shape may be assumed.

The image creator 26 reduces the respective body part sizes calculated as described above respectively on the display 21 to the sizes which allow the dot-matrix display and creates a body image of a human shape.

First of all, as illustrated in FIGs. 9A and 9B, the image creator 26 determines the body trunk shape in the front viewed from the Y direction (FIG. 9A) and the side surface viewed from the X direction (FIG. 9B) respectively on the basis of the body trunk length calculated as described above(see Step S202), the acromial width G, the infra-axillary width H, the waist height J, the navel height I, the long axis of abdominal circumference 2x, the abdominal short axis 2y, and the infra-axillary length (see Step S207) . The smoothing process smoothes the body trunk shape by, for example, setting lines connecting predetermined points such as lines connecting both ends of the long axis of abdominal circumference 2x and both ends of the infra-axillary width H respectively (alternate long and short dashed lines in FIG. 9A), lines connecting both ends of the acromial width G and the both ends of the infra-axillary width H respectively (alternate long and two short dashed lines in FIG. 9A), lines connecting both ends of the abdominal short axis 2y and both ends of the infra-axillary thickness respectively (alternate long and short dashed line in FIG. 9B), correcting an outline of the body image of the body trunk shape so as to extend along the lines.

The image creator 26 determines the leg shape on the basis of the leg length B and the leg diameter (see Step S204, R2× 2), and determines the arm shape on the basis of the arm length C and the arm diameter (see Step S204, R2×2). The shapes of the lean body of the legs are each determined on the basis of the leg length B and the diameter of the lean body of the leg (R1× 2), the shapes of the lean body of the arms are overlapped with the determined leg shapes by aligning the centers, and body image indicating portions which are not overlapped as fat (hatched in FIGs. 9A and 9B) is created. In the same manner, the shapes of the lean body of the arms are each determined on the basis of the arm length C and the diameter of lean body of the arm (R1× 2), the shapes of the lean body of the legs are overlapped with the determined arm shape by aligning the centers, and body image indicating portions which are not overlapped as fat (hatched in FIGs. 9A and 9B) are created. In this manner, the body image differentiating between fat and lean body of the body parts of the user is preferable because the user can easily confirm the ratio through instinct. Since many of the users who diet are interested in a point to slim down the arms or the legs, being capable of confirming the ratio of fat with respect to the lean body of the arms or the legs visually is specifically effective for those who diet. In this embodiment, an example of creating the body image differentiating between fat and lean body of the legs and arms has been described. However, the invention is not limited thereto, and it is also possible to configure the apparatus so as to create a body image in which fat and lean body of only one of the legs and arms are differentiated, or configure the apparatus so as to create the body image in which fat and lean body of other body parts are differentiated may be created.

The image creator 26 further determines the head shape on the basis of the head width D, the head length E, and the fore-and-aft head length F.

The image creator 26 combines the body image of the leg, the body image of the arm, the body image of the head, and the body image of the body trunk created in Step 209 and creates an body image indicating the entire figure of the user (Step S210) . More specifically, the image creator 26 creates the body image of the front of the user as illustrated in FIG. 9A, and/or a body image of the side surface of the user as illustrated in FIG. 9B.

As illustrated in FIG. 9B, when creating the body image of the side surface, the image creator 26 combines a body image of the body trunk and the body image of the arm on the basis of the length L between the back to the center portion of the arm calculated in Step S208. Also, when combining the body image of the body trunk and the body image of the head, the image creator 26 provides a gap depending on the neck length calculated in Step S205 or placing the body image interposed therebetween.

FIGs. 9A and 9B are schematic drawings for explaining a technology to create the image body by the image creator 26. However, as illustrated in FIGs. 10A and 10B, creating an image body higher in resolution is preferable. In FIGs. 10A and 10B, "FV" is a body image viewed from the front, and "SV" is a body image viewed from the side surface.

FIG. 10A is an example of a body image of the user having a standard figure, in which the amounts of fat of the legs and the arms is relatively small and hence the displayed fat (hatched portion) is thin. In contrast,

FIG. 10B is an example of a body image of an overweighed user, in which the fat is large on the legs and arms, and hence the displayed fat (hatched portion) is thick. It is needless to say that the resolution may be further enhanced in comparison with the example of the body images illustrated in FIGs. 10A and 10B, or the smoothing process is further performed to create a body image with a good appearance.

In the configuration as described above, the following advantages are achieved according to the embodiment.

(1) the user is capable of figuring out the state of the entire body or the respective body parts through instinct by the display of the body image, whereby the user is capable of making synthetic determination relating to the diet or the like, so that the motivation of the diet in the future may be enhanced.

(2) By confirming the body image using the biometric apparatus of the invention on the daily basis, the user is capable of understanding his or her figure quickly and easily in an objective way. Accordingly, the consciousness of the figure of his or her own may be obtained further in detail.

(3) In particular, since the body image differentiating between the fat and the lean body of the body parts of the user may be displayed, the user can easily confirm the ratio through instinct than the display of the result of measurement only by the numerical value in the apparatus of the related art.

(4) The user only has to input characteristic information of the individual used commonly used such as height, sex, age, in the initial setting in the same manner as the weighing machine with body composition meter of the related art, and the measuring operation to be performed is the same as that of the apparatus of the related art, so that the user is not forced to do the specific operation more than that of the related art.

A modification of the first embodiment will be described below.

(Modification 1)

In the size calculating process in Step S201 to Step S208 in FIG. 4, an example in which the arithmetic section 24 calculates the body part sizes has been described. However, the body part sizes are not necessarily limited to be calculated by the arithmetic section 24 using the input biometric information or the measured biometric information (body composition information). For example, the user inputs the measured values acquired by a measure, a CT (Computer laminagraphy), an abdominal adipometer, a subcutaneous fat meter and other units using the operating unit 22, and the image creator 26 uses the input measured values to execute the image creating process (Step S209 and Step S210).

(Modification 2)

The image creator 26 may be configured to correct a model data relating to the figure stored in the storage 25 on the basis of the body part size calculated by the arithmetic section 24 and create the body image.

It Step S209 in FIG. 4 described above, the image creating process assuming the simple model has been described. Alternatively, the image creating process as described below may be performed.

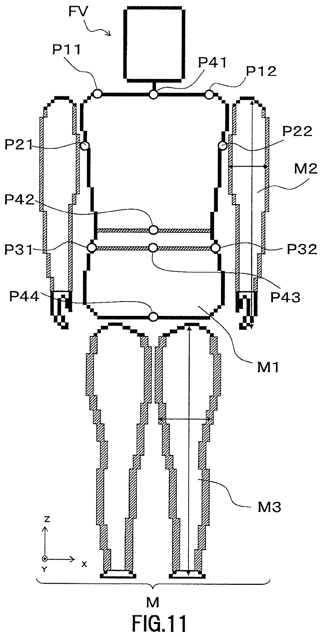

FIG. 11 illustrates an example of a basic model used for determining the shapes of body trunk, arms, and legs. A basic model M illustrated in FIG. 11 is a model data imitating a standard figure, and includes a body trunk model M1, an arm model M2 and a leg model M3. The image creator 26 is configured to correct the shapes of the body trunk model M1, the arm model M2, and the leg model M3 according to the body part sizes calculated as described above, and create a body image of the user. As illustrated in FIG. 11, the basic model M includes a natural concave-convex shape of the human body in the initial state, and the shape (specifically, the ratio of vertical and horizontal sizes) is corrected on the basis of the body part sizes calculated by the arithmetic section 24 as an extension of the model data as described above, so that the body image having the concave and convex shape closer to the human body may be created easily.

More specifically, the process of determination of the body trunk shape using the basic model M in FIG. 11 executes the processes from (a) to (g) described below as an example.

(a) On the basis of the ratio of the body trunk length with respect to the length under neck A, the distance and the position between points P41 and P44 of the model M1 is determined.

(b) On the basis of the waist height J, the position (height) of a point P42 of the model M1 is determined.

(c) On the basis of the navel height I, the position (height) of points P43, P31, and P32 of the model M1 is determined.

(d) Distances between points P11 and P12, between points P21 and P22, and between the points P31 and P32 of the model M1 are obtained respectively on the basis of the ratio of the acromial width G with respect to the body trunk length, the ratio of the infra-axillary width H with respect to the body trunk length, and the ratio of the long axis of abdominal circumference 2x with respect to the body trunk length.

(e) On the basis of the ratio of the leg length B with respect to the length under neck A, length of the model M3 is determined. The thickness of the model M3 is also determined on the basis of the diameter of the leg (see Step S204, R2×2) with respect to the leg length B.

(f) The length of the model M2 is determined on the basis of the arm length C with respect to the length under neck A or the height. The thickness of the model M2 is also determined on the basis of the diameter of the arm (see Step S204, R2×2) with respect to the arm length C.

(g) The body trunk model M1 (one), the arm models M2 (two in lateral symmetry), and the leg model M3 (two in lateral symmetry) are combined to create the body image.

The processes of (a) to (g) described above are performed by the arithmetic section 24 and the image creator 26 according to the instruction from the controller 29. The processes (a) to (f) may be executed in the order other than that described above. The head portion may be processed in the same manner as the above-described body trunk, the arms, and the legs. In addition, FIG. 11 illustrates a body image viewed from the front. However, the body image of the side surface may be created in the same manner as described above.

Here, the procedure which provides the above-described model with a bias by the fat percentage may be employed. For example, as the procedure which lowers the center of gravity, a process such as lowering the positions of the maximum diameter of the lower legs in the leg model M3 is added.

Depending on whether the visceral fat pyknic type or the subcutaneous fat pyknic type, the body trunk model M1 is corrected. For example, the shape of the abdominal circumference of the visceral fat pyknic type is closer to a true circle in comparison with the subcutaneous fat pyknic type, and consequently, in the body image of the body trunk described later, the abdominal portion is formed to develop to the front.

(Modification 3)

The body image created in Steps S209 and S210 in FIG. 4 is not limited to a two-dimensional image as illustrated in FIGs. 9A and 9B and FIGs. 10A and 10B, and a three-dimensional body image in which the fat mass, the muscle mass, and the texture or the like are reflected on the basis of the data calculated for the biometric information measured by the load cell 27 and the biometric impedance measuring unit 28, the biometric information input by the user, and data calculated from these items of biometric information may be created. For example, an image is added with texture such that (1) if the body trunk includes a little subcutaneous fat but much of visceral fat, the abdominal portion is formed to develop to the front, (2) if much subcutaneous fat is included, a figure protruded to the left and right is formed, (3) the older the user gets, the more the fat glows down, (4) if the muscle mass is large, building muscles are expressed. The three-dimensional image is preferably configured to be rotatable for allowing the user to view the body image from any direction.

(Modification 4)

In the embodiment described above, the example in which the body image indicating the entire body shape including legs, arms, head, and body trunk has been described. However, a body image indicating the figure of only a part of the body (for example, a body image indicating the shape of the body trunk) may be created and displayed.

(Modification 5)

In the embodiment described above, the example in which the body images in which fat is differentiated from lean body are created and displayed has been described. However, it is needless to say that body images without such differentiation may be used. It is also possible to allow the user to select whether the body image in which fat is differentiated from lean body is to be displayed or the body image in which fat is not differentiated from the lean body is to be displayed.

(Second Embodiment)

A second embodiment of the invention will be described.

The second embodiment is configured as a computer-readable storage medium storing a body image creating program that causes an information processing apparatus to execute the size calculating process (Step S201 to S208) and the image creating process (Step S209 to S210) illustrated in FIG. 4. Accordingly, the biometric system including the information processing apparatus in which a computer-readable storage medium storing the body image creating program is installed and the body composition meter configured to measure the biometric information to be input the information processing apparatus is configured. Here, the size calculating process is a process performed by an arithmetic section provided in the information processing apparatus for calculating the body part sizes of the user on the basis of the biometric information input into the information processing apparatus, and the image creating process is a process performed by a control unit provided in the information processing apparatus for creating the body image of the user on the basis of the body part sizes calculated by the size calculating process.

The body image creating program is configured to cause a display unit of the information processing apparatus to display the body image created by the image creating process.

The information processing apparatus includes mobile computers such as mobile phone sets and smart phones in addition to personal computers and, in the case of the mobile phones sets or the smart phones, the body image creating program of this embodiment is preferably provided as an application downloadable through a website.

According to this embodiment, the user needs not to buy the biometric apparatus as in the first embodiment newly if the body image creating program of this embodiment is installed in a computer-readable storage medium of the user's own information processing apparatus, and is allowed to create and display a body image by the information processing apparatus by measuring his or her own biometric information using a general existing body composition meter and inputting results of measurement and other required items of biometric information into the information processing apparatus.

The biometric information measured by the body composition meter here may be input manually into the information processing apparatus, but may be input to the information processing apparatus via existing communication devices or storage media.

Modifications of the second embodiment will be described.

(Modification 1)

Preferably, the body image to be displayed is a three-dimensional image, and a display process that allows the user to rotate the body image and display the body image from any arbitrary direction by the operation of the information processing apparatus is executed.

The display of rotation of the body image is preferably a display process which allows to be performed by pinching or flicking operation in the case of the information processing apparatus having a multi-touch interface (in particular, the mobile computers such as smart phones) . The pinching operation is an operation to place two fingers on the display surface with a touch-panel function and move these fingers toward each other so as to pinch the display (pinch-in), or move these fingers away from each other (pinch-out), and the flicking operation is an operation to swipe the display with a fingertip.

(Modification 2)

The body image creating program may be configured to cause the information processing apparatus to execute a target setting process for setting the body image of a target figure of the user by modifying the body image by expanding or contracting the entire or part of the body image by the pinching operation as described above . The body image creating program may be configured to cause the information processing apparatus to execute the target setting process for setting the texture of the body image of the target figure of the user (for example, how much muscled figure is wanted, that is, the target fat mass) . The body image creating program may be configured to cause the information processing apparatus to execute the target setting process for calculating the amount of change in body composition required for attaining the target figure, for example, the numerical values of the visceral fat, the subcutaneous fat, the increase and decrease of muscle and presenting the calculated values when the body image of the target figure is set by changing the body image as described above.

The body image creating program may be configured to cause the information processing apparatus to execute the target setting process which is capable of displaying the body image corresponding to a target value when the user inputs the target value (numerical values) such as the body composition. Accordingly, the user is allowed to figure out the image that the user would be when the target is attained through instinct. In order to allow the user to visually confirm the degree of target attainment, the body image creating program may be configured to cause the information processing apparatus to execute a display process that displays the comparison between the body image of the target figure set in the past and the current body image.

(Third Embodiment)

A third embodiment of the invention will be described.

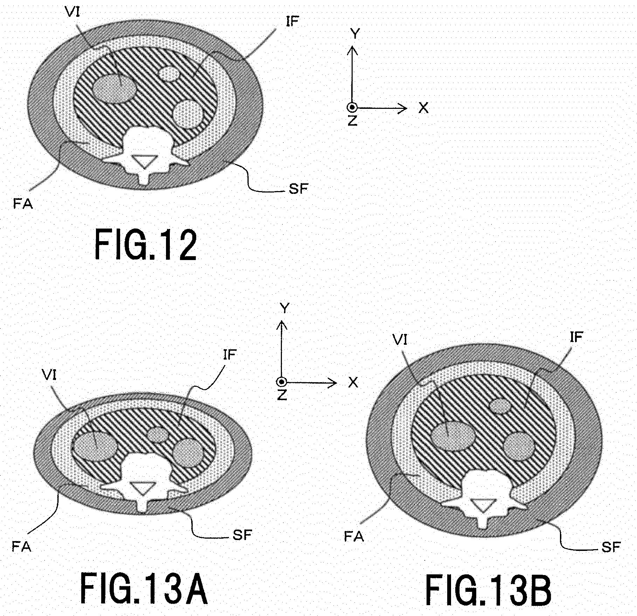

In the biometric apparatus of the third embodiment, a body image of an abdominal cross-section of the user may be formed. FIG. 12 illustrates an example of a body image imitating an abdominal cross-section of the user taken along the direction orthogonal to the Z direction. In FIG. 12 (and FIGs. 13A and 13B described later), a subcutaneous fat SF, a fascia FA, a viscera VI, and a visceral fat IF are illustrated. FIG. 13A illustrates an example of the body image imitating the abdominal cross-section of the user having less subcutaneous fat and visceral fat than standard, and FIG. 13B illustrates an example of the body image imitating the abdominal cross-section of the user having larger subcutaneous fat and visceral fat than standard.

The cross section of a body part such as the abdominal portion may be created and displayed as a mode of the body image of the invention. A simple model in which the shape of the abdominal circumference cross-section and the shape of the visceral fat area are both an ellipsoidal shape is assumed.

First of all, an ellipsoidal shape as a model of the abdominal circumference cross-section is determined. The half long axis of abdominal circumference x and the half short axis y of the abdominal circumference are calculated using the following expressions (6) and (9).

$$x = (\beta - \sqrt{(\beta^2 - 4\alpha \cdot K)}) / 2\alpha \quad \cdots \quad (6)$$

$$y = Q \cdot Q'_{VFA} \cdot x \quad \cdots \quad (9)$$

where

$$\alpha = (1 - Q)^2 / 2.2$$
$$\beta = \pi \sqrt{(2(1 + Q^2))}$$

K is an abdominal circumference, and may be calculated in the same manner as Step S201. Q is a vertical and horizontal ratio of the abdominal circumference, and a correspondence relation compiled in a database is used (see FIG. 5). $Q'_{VFA}$ is a coefficient according to a visceral fat area (VFA) and is set so that an ellipticity of the cross-sectional shape of the abdominal circumference is lowered as the visceral fat area increases.

Subsequently, the elliptical shape as a model of the visceral fat area is determined. The visceral fat area is already calculated in the Step S106. The surface area of the ellipse is obtained by

$$\pi \times \text{half long axis} \times \text{half short axis,}$$

and hence the visceral fat area VFA is obtained by the estimated expression (10)

$$VFA = \pi \cdot y' \cdot x' = \pi \cdot Q \cdot Q'_{VFA} \cdot x'^2 \quad \cdots \quad (10)$$

where x is a half long axis of the visceral fat area, y'Q·Q'VFA·s' is a half short axis of the visceral fat area.

On the basis of the expression (10), the half long axis x' is calculated by the following expression (11).

$$x' = \sqrt{(VFA / \pi \cdot Q \cdot Q'_{VFA})} \quad \cdots \quad (11)$$

The thickness of the fascia FA and the size of the viscera VI are determined on the basis of the muscle mass of the body trunk. The muscle mass of the body trunk may be calculated by scores of 9 levels, for example, in Step S106, and the thickness of the fascia FA and the size of the viscera VI are determined according to the calculated score.

The image creator 26 creates a body image imitating the abdominal cross-section of the user abdominal portion by arranging the ellipse as a model of the abdominal circumference cross-section (the ellipsoidal model of the abdominal circumference cross-section), the ellipse as a model of the visceral fat area (the ellipsoidal model of the visceral fat area), the fascia FA, and the viscera VI determined in shape as described above and adding a shape of a backbone at a lower center as illustrated in FIG. 12 and FIGs. 13A and 13B. In the case of the standard figure of the user, the body image as illustrated in FIG. 12 is created on the basis of the biometric information, and if the amounts of subcutaneous fat and visceral fat are less than that of the standard, the body image as illustrated in FIG. 13A is created, and if the amounts of subcutaneous fat and the visceral fat are more than the standard, the body image as illustrated in Fig. 13B is created.

The biometric apparatus of the third embodiment is the same as the weighing machine 10 of the first embodiment other than the point that the body image of the abdominal cross-section of the user may be created as described above.

Alternatively, in the biometric apparatus of the third embodiment, the body image imitating the cross section of the body part as described above are created and displayed in addition to the body image indicating the entire part of the figure of the user's body that the weighing machine 10 according to the first embodiment creates.

According to the biometric apparatus of the third embodiment, the schematic body image of the abdominal cross-section can be displayed easily on the basis of the user's current biometric information without depending on the apparatus such as CT machine used in the medical facilities, so that the user can have the consciousness relating to the user's own figure for further in detail.

Although the invention has been described on the basis of the above-described embodiment, the invention is not limited to the above-described embodiment, and may be improved or modified within the scope of the claims.

Industrial Applicability

As described above, the biometric apparatus of the invention is effective for obtaining the consciousness relating to the user's own figure in detail and objectively.

**Claims**

1. A biometric apparatus (10) comprising:

   a biometric information acquiring unit (22, 24, 28, 29) configured to acquire biometric information of a user, wherein the biometric information acquired by the biometric information acquiring unit (22, 24, 28, 29) includes height, sex, and age of the user, and body composition information of the entire body and on a part-to-part basis of different body parts;
   a storing unit (25) configured to store a correspondence relation having a ratio of lengths of the body parts with respect to the height, sex and age;
   an arithmetic operation unit (24) configured to calculate lengths of the body parts of the user on the basis of height, sex, and age of the user acquired by the biometric information acquiring unit (22, 24, 28, 29) and the correspondence relation stored in the storing unit (25), wherein the arithmetic operation unit (24) is further configured to calculate first diameters (R1) of lean body of arms and legs, and second diameters (R2) of fat body and lean body of arms and legs on the basis of the lengths of the body parts and the body composition information of the body parts;
   an image creating unit (26) configured to create a body image in which fat body and lean body of the arms and legs are differentiated on the basis of diameters and lengths of the arms and legs; and
   a display unit (21) configured to display the body image created by the image creating unit (26), wherein the body image displayed on the display includes an image indicating the entire figure of the user's body, the entire figure of the user's body including differentiating fat body and lean body of the arms and differentiating fat body and lean body of the legs.

2. The biometric apparatus (10) according to Claim 1, wherein the image creating unit (26) creates the body image by correcting a model data relating to a figure stored in the storing unit (25) on the basis of the diameters and lengths of the arms and legs.

3. The biometric apparatus (10) according to any one of Claims 1 or 2, wherein the arithmetic operation unit (24) calculates the abdominal circumference, the length of the body trunk, the lengths of the arms, the lengths of the legs, fat volumes and lean body volumes of the respective body parts, the average cross-sectional area of the fat and the average cross-sectional area of the lean body of the respective body parts, the arm diameters, the leg diameters, the acromial width, the infra-axillary width, the navel height, the waist height, and the long axis of abdominal circumference, and the image creating unit (26) creates an body image of the front of the user on the basis of the abdominal circumference, the length of the body trunk, the lengths of the arms, the lengths of the legs, fat volumes and lean body volumes of the respective body parts, the average cross-sectional area of the fat and the average cross-sectional area of the lean body of the respective body parts, the arm diameters, the leg diameters, the acromial width, the infra-axillary width, the navel height, the waist height, and the long axis of abdominal circumference.

4. The biometric apparatus (10) according to Claim 3, wherein the arithmetic operation unit (24) calculates the infra-axillary thickness, the short axis of abdominal circumference, and the length from the back to the centers of the arms and the image creating unit creates a body image of the side surface of the user on the basis of the infra-axillary thickness, the short axis of abdominal circumference, and the length from the back to the centers of the arms.

**5.** The biometric apparatus (10) according to any one of Claims 1 to 4, wherein the image creating unit (26) creates a body image imitating the cross section of the body parts of the user, which is a body image differentiating fat and lean body.

**6.** The biometric apparatus (10) according to Claim 5, wherein the image creating unit (26) creates a body image imitating an abdominal cross-section of the user including an ellipsoidal model of an abdominal circumference cross-section of the user and an ellipsoidal model of the visceral fat area of the user.

**7.** A computer-readable storage medium storing a body image creating program executed by an information processing apparatus capable of receiving biometric information of a user from a biometric information acquiring unit (22, 24, 28, 29) for creating a body image of the user on the basis of the biometric information, wherein the biometric information includes height, sex, and age of the user, and body composition information of the entire body and on a part-to-part basis of different body parts, and wherein
the program causes the information processing apparatus to execute;
a size calculating process for calculating lengths of the body parts of the user on the basis of height, sex, and age of the user and a correspondence relation having a ratio of lengths of the body parts with respect to the height, sex and age, and for calculating first diameters (R1) of lean body of arms and legs, and second diameters (R2) of fat body and lean body of arms and legs on the basis of the lengths of the body parts and the body composition information of the body parts,
an image creating process for creating a body image in which fat body and lean body of the arms and legs are differentiated on the basis of the diameters and lengths of the arms and legs; and
a display process for displaying the body image created by the image creating process on a display, wherein the body image displayed on the display includes an image indicating the entire figure of the user's body, the entire figure of the user's body including differentiating fat body and lean body of the arms and differentiating fat body and lean body of the legs.

**8.** A computer-readable storage medium according to Claim 7, wherein the information processing apparatus comprises a computer or a mobile computer.

**Patentansprüche**

**1.** Biometrische Vorrichtung (10), umfassend:

eine Biometrische-Informationen-Erfassungseinheit (22, 24, 28, 29), die konfiguriert ist zum Erfassen von biometrischen Informationen eines Benutzers, wobei die durch die Biometrische-Informationen-Erfassungseinheit (22, 24, 28, 29) erfassten biometrischen Informationen die Körpergröße, das Geschlecht und das Alter des Benutzers sowie Köperzusammensetzungsinformationen des gesamten Körpers und auf einer Teil-für-Teil-Basis von verschiedenen Körperteilen umfassen,
eine Speichereinheit (25), die konfiguriert ist zum Speichern einer Korrespondenzbeziehung mit einem Längenverhältnis der Körperteile in Bezug auf die Körpergröße, das Geschlecht und das Alter,
eine arithmetische Operationseinheit (24), die konfiguriert ist zum Berechnen von Längen der Körperteile des Benutzers basierend auf der Körpergröße, dem Geschlecht und dem Alter des Benutzers, die durch die Biometrische-Informationen-Erfassungseinheit (22, 24, 28, 29) erfasst wurden, und der in der Speichereinheit (25) gespeicherten Korrespondenzbeziehung, wobei die arithmetische Operationseinheit (24) weiterhin konfiguriert ist zum Berechnen von ersten Durchmessern (R1) von Armen und Beinen eines schlanken Körpers und von zweiten Durchmessern (R2) von Armen und Beinen eines dicken Körpers und eines schlanken Körpers basierend auf den Längen der Körperteile und den Körperzusammensetzungsinformationen der Körperteile,
eine Bilderzeugungseinheit (26), die konfiguriert ist zum Erzeugen eines Körperbilds, in dem die Arme und die Beine eines dicken Körpers und eines schlanken Körpers basierend auf den Durchmessern und den Längen der Arme und Beine differenziert werden, und
eine Anzeigeeinheit (21), die konfiguriert ist zum Anzeigen des durch die Bilderzeugungseinheit (26) erzeugten Körperbilds, wobei das an der Anzeige angezeigte Körperbild ein Bild umfasst, das die gesamte Figur des Körpers des Benutzers angibt, wobei in der gesamten Figur des Körpers des Benutzers die Arme des dicken Körpers und des schlanken Körpers differenziert werden und die Beine des dicken Körpers und des schlanken Körpers differenziert werden.

**2.** Biometrische Vorrichtung (10) nach Anspruch 1, wobei die Bilderzeugungseinheit (26) das Körperbild erzeugt, indem sie in der Speichereinheit (25) gespeicherte Modelldaten zu einer Figur basierend auf den Durchmessern und Längen der Arme und Beine korrigiert.

3. Biometrische Vorrichtung (10) nach Anspruch 1 oder 2, wobei die arithmetische Operationseinheit (24) den Unterleibsumfang, die Länge des Körperrumpfs, die Länge der Arme, die Länge der Beine, dicke Körpervolumen und schlanke Körpervolumen der entsprechenden Körperteile, die durchschnittliche Querschnittfläche für einen dicken Körper und die durchschnittliche Querschnittfläche für einen schlanken Körper der entsprechenden Körperteile, die Armdurchmesser, die Beindurchmesser, die Schulterdachbreite, die Achselbreite, die Nabelhöhe, die Taillenhöhe und die lange Achse des Unterleibumfangs berechnet und die Bilderzeugungseinheit (26) ein Körperbild der Vorderseite des Benutzers basierend auf dem Unterleibsumfang, der Länge des Körperrumpfs, der Längen der Arme, der Längen der Beine, der dicken Körpervolumen und der schlanken Körpervolumen der entsprechenden Körperteile, der durchschnittlichen Querschnittfläche für einen dicken Körper und der durchschnittlichen Querschnittfläche für einen schlanken Körper der entsprechenden Körperteile, der Armdurchmesser, der Beindurchmesser, der Schulterdachbreite, der Achselbreite, der Nabelhöhe, der Taillenhöhe und der langen Achse des Unterleibumfangs erzeugt.

4. Biometrische Vorrichtung (10) nach Anspruch 3, wobei die arithmetische Operationseinheit (24) die Achseldicke, die kurze Achse des Unterleibumfangs und die Länge vom Rücken zu den Mitten der Arme berechnet und die Bilderzeugungseinheit ein Körperbild der Seitenfläche des Benutzers basierend auf der Achselbreite, der kurzen Achse des Unterleibumfangs und der Länge vom Rücken zu den Mitten der Arme erzeugt.

5. Biometrische Vorrichtung (10) nach einem der Ansprüche 1 bis 4, wobei die Bilderzeugungseinheit (26) ein Körperbild erzeugt, das den Querschnitt der Körperteile des Benutzers imitiert und ein zwischen einem dicken und einem schlanken Körper differenzierendes Körperbild ist.

6. Biometrische Vorrichtung (10) nach Anspruch 5, wobei die Bilderzeugungseinheit (26) ein Körperbild erzeugt, das einen Unterleibsquerschnitt des Benutzers einschließlich eines ellipsenförmigen Modells eines Unterleibsumfangsquerschnitts des Benutzers und eines ellipsenförmigen Modells des Viszeralfettbereichs des Benutzers imitiert.

7. Computerlesbares Speichermedium, das ein Körperbild-Erzeugungsprogramm speichert, das durch eine Informationsverarbeitungsvorrichtung ausgeführt wird, die biometrische Informationen eines Benutzers von einer Biometrische-Informationen-Erfassungseinheit (22, 24, 28, 29) empfangen kann, um ein Körperbild des Benutzers basierend auf den biometrischen Informationen zu erzeugen, wobei die biometrischen Informationen die Körpergröße, das Geschlecht und das Alter des Benutzers sowie Körperzusammensetzungsinformationen des gesamten Körpers und auf einer Teil-für-Teil-Basis von verschiedenen Körperteilen umfassen,
wobei das Programm veranlasst, dass die Informationsverarbeitungsvorrichtung ausführt:

einen Größenberechnungsprozess zum Berechnen der Längen der Körperteile des Benutzers basierend auf der Körpergröße, dem Geschlecht und dem Alter des Benutzers und einer Korrespondenzbeziehung mit einem Längenverhältnis der Körperteile in Bezug auf die Körpergröße, das Geschlecht und das Alter, und zum Berechnen von ersten Durchmessern (R1) von Armen und Beinen eines schlanken Körpers und von zweiten Durchmessern (R2) von Armen und Beinen eines dicken Körpers und eines schlanken Körpers basierend auf den Längen der Körperteile und den Körperzusammensetzungsinformationen der Körperteile,
einen Bilderzeugungsprozess zum Erzeugen eines Körperbilds, in dem die Arme und die Beine eines dicken Körpers und eines schlanken Körpers basierend auf den Durchmessern und den Längen der Arme und Beine differenziert werden, und
einen Anzeigeprozess zum Anzeigen des durch den Bilderzeugungsprozess erzeugten Körperbilds an einer Anzeige, wobei das an der Anzeige angezeigte Körperbild ein Bild umfasst, das die gesamte Figur des Körpers des Benutzers angibt, wobei in der gesamten Figur des Körpers des Benutzers die Arme des dicken Körpers und des schlanken Körpers differenziert werden und die Beine des dicken Körpers und des schlanken Körpers differenziert werden.

8. Computerlesbares Speichermedium nach Anspruch 7, wobei die Informationsverarbeitungsvorrichtung ein Computer oder ein mobiler Computer ist.

**Revendications**

1. Appareil biométrique (10) comprenant :

   une unité d'acquisition d'information biométrique (22, 24, 28, 29) configurée pour acquérir de l'information biométrique sur un utilisateur, l'information biométrique acquise par l'unité d'acquisition d'information biométrique (22, 24, 28, 29) comprenant la taille, le sexe, et l'âge de l'utilisateur, et de l'information de composition du corps sur le corps entier et sur une base de partie-à-partie de différentes parties corporelles ;
   une unité de stockage (25) configurée pour stocker une relation de correspondance présentant un rapport des longueurs des parties corporelles par rapport à la taille, au sexe et à l'âge ;
   une unité d'opération arithmétique (24) configurée pour calculer les longueurs des parties corporelles de l'utilisateur sur la base de la taille, du sexe, et de l'âge de l'utilisateur acquises par l'unité d'acquisition d'information biométrique (22, 24, 28, 29) et de la relation de correspondance stockée dans l'unité de stockage (25), l'unité d'opération arithmétique (24) étant en outre configurée pour calculer les premiers diamètres (R1) de corps mince des bras et des jambes, et les seconds diamètres (R2) de corps gros et de corps mince des bras et des jambes sur la base des longueurs des parties corporelles et de l'information de composition du corps des parties corporelles ;
   une unité de création d'image (26) configurée pour créer une image corporelle dans laquelle le corps gros et le corps mince des bras et des jambes sont différenciés sur la base des diamètres et des longueurs des bras et des jambes ; et
   une unité d'affichage (21) configurée pour afficher l'image corporelle créée par l'unité de création d'image (26), l'image corporelle affichée sur l'affichage comprenant une image indiquant l'illustration entière du corps de l'utilisateur, l'illustration entière du corps de l'utilisateur comprenant la différenciation de corps gros et de corps mince des bras et la différenciation de corps gros et de corps mince des jambes.

2. Appareil biométrique (10) selon la revendication 1, l'unité de création d'image (26) créant l'image corporelle par correction des données de modèle concernant une illustration stockée dans l'unité de stockage (25) sur la base des diamètres et des longueurs des bras et des jambes.

3. Appareil biométrique (10) selon l'une quelconque des revendications 1 ou 2, l'unité d'opération arithmétique (24) calculant la circonférence abdominale, la longueur du tronc du corps, les longueurs des bras, les longueurs des jambes, les volumes de graisse et les volumes de corps mince des parties corporelles respectives, la surface transversale moyenne de corps gros et la surface transversale moyenne de corps mince des parties corporelles respectives, les diamètres des bras, les diamètres des jambes, la largeur acromiale, la largeur infra-axillaire, la hauteur du nombril, la hauteur de la ceinture, et l'axe long de la circonférence abdominale, et l'unité de création d'image (26) créant une image corporelle de l'avant de l'utilisateur sur la base de la circonférence abdominale, de la longueur du tronc du corps, des longueurs des bras, des longueurs des jambes, des volumes de graisse et des volumes de corps mince des parties corporelles respectives, la surface transversale moyenne de corps gros et la surface transversale moyenne de corps mince des parties corporelles respectives, les diamètres des bras, les diamètres des jambes, la largeur acromiale, la largeur infra-axillaire, la hauteur du nombril, la hauteur de la ceinture, et l'axe long de la circonférence abdominale.

4. Appareil biométrique (10) selon la revendication 3, l'unité d'opération arithmétique (24) calculant l'épaisseur infra-axillaire, l'axe court de la circonférence abdominale, et la longueur depuis l'arrière vers les centres des bras et l'unité de création d'image créant une image corporelle de la surface latérale de l'utilisateur sur la base de l'épaisseur infra-axillaire, l'axe court de la circonférence abdominale, et la longueur depuis l'arrière vers les centres des bras.

5. Appareil biométrique (10) selon l'une quelconque des revendications 1 à 4, l'unité de création d'image (26) créant une image corporelle imitant la section transversale des parties corporelles de l'utilisateur, qui est une image corporelle effectuant une différenciation entre corps gros et corps mince.

6. Appareil biométrique (10) selon la revendication 5, l'unité de création d'image (26) créant une image corporelle imitant une section abdominale de l'utilisateur comprenant un modèle ellipsoïdal d'une section transversale de circonférence abdominale de l'utilisateur et un modèle ellipsoïdal de la surface de graisse viscérale de l'utilisateur.

7. Milieu de stockage pouvant être lu par ordinateur stockant un programme de création d'image corporelle exécuté par un appareil de traitement d'information capable de recevoir l'information biométrique d'un utilisateur provenant d'une unité d'acquisition d'information biométrique (22, 24, 28, 29) afin de créer une image corporelle de l'utilisateur sur la base de l'information biométrique, l'information biométrique comprenant la taille, le sexe, et l'âge de l'utilisateur, et l'information de composition du corps du corps entier et sur une base de partie-à-partie des différentes parties corporelles, et

le programme amenant l'appareil de traitement d'information à exécuter ;

un processus de calcul de taille pour calculer les longueurs des parties corporelles de l'utilisateur sur la base de la taille, du sexe, et de l'âge de l'utilisateur et une relation de correspondance présentant un rapport des longueurs des parties corporelles par rapport à la taille, au sexe et à l'âge, et afin de calculer les premiers diamètres (R1) de corps mince des bras et des jambes, et les seconds diamètres (R2) de corps gros et de corps mince des bras et des jambes sur la base des longueurs des parties corporelles et de l'information de composition du corps des parties corporelles,

procédé de création d'image destiné à créer une image corporelle dans laquelle un corps gros et un corps mince des bras et des jambes sont différenciés sur la base des diamètres et des longueurs des bras et des jambes ; et

procédé d'affichage permettant d'afficher l'image corporelle créée par le procédé de création d'image sur un affichage, l'image corporelle affichée sur l'affichage comprenant une image indiquant l'illustration entière du corps de l'utilisateur, l'illustration entière du corps de l'utilisateur comprenant la différenciation de corps gros et de corps mince des bras et la différenciation de corps gros et de corps mince des jambes.

8. Milieu de stockage pouvant être lu par ordinateur selon la revendication 7, l'appareil de traitement d'information comprenant un ordinateur ou un ordinateur portable.

130

130L

131a

21

22

131b

132a

127

130R

132b

128

20

31a

31b

23

32a

32b

10

# FIG.1

| 21 | DISPLAY | | CONTROLLER | | STORAGE | 25 |
| 22 | OPERATING UNIT | | | | IMAGE CREATOR | 26 |
| 23 | FOOT SWITCHES | | | | LOAD CELL | 27 |
| 24 | ARITHMETIC SECTION | | | | BIOMETRIC IMPEDANCE MEASURING UNIT | 28 |

29

# FIG.2

START

S101 — SET KEY OPERATION DONE? —— Yes

No

S103 — LOAD CELL ZERO-POINT UPDATE PROCESS

S104 — WEIGHT MEASURING PROCESS

S105 — BIOMETRIC IMPEDANCE MEASURING PROCESS

S106 — BIOMETRIC INFORMATION CALCULATING PROCESS

S107 — SIZE CALCULATING·IMAGE CREATING PROCESS

S108 — RESULT DISPLAY PROCESS

END

INITIAL SETTING PROCESS

S102

END

FIG.3

**S106**

↓

S201 ～ | CALCULATE ABDOMINAL CIRCUMFERENCE

↓

S202 ～ | CALCULATE LENGTHS OF BODY TRUNK, LEGS, AND ARMS

↓

S203 ～ | CALCULATE VOLUME AND AVERAGE CROSS-SECTIONAL AREAS OF RESPECTIVE BODY PARTS

↓

S204 ～ | CALCULATE DIAMETERS OF LEGS AND DIAMETERS OF ARMS

↓

S205 ～ | CALCULATE HEAD WIDTH, HEAD LENGTH, FORE-AND-AFT HEAD LENGTH, NECK LENGTH, ACROMIAL WIDTH, INFRA-AXILLARY WIDTH, NAVEL HEIGHT, AND WAIST HEIGHT

↓

S206 ～ | CALCULATE LONG AXIS (2x) AND SHORT AXIS (2y) OF ABDOMINAL CIRCUMFERENCE

↓

S207 ～ | CALCULATE INFRA-AXILLARY THICKNESS

↓

S208 ～ | CALCULATE LENGTH OF BACK TO CENTER PORTION OF ARM

↓

S209 ～ | DETERMINE THE SHAPES OF RESPECTIVE BODY PARTS AND SMOOTHENING PROCESS

↓

S210 ～ | COMBINE LEG, ARM, HEAD, AND BODY TRUNK

**S107**

↓

**S108**

# FIG.4

| | HEIGHT | A<br>UNDER<br>NECK | B<br>LEG | C<br>ARM | D<br>HEAD<br>WIDTH | E<br>HEAD<br>LENGTH | F<br>FORE-<br>AND-AFT<br>HEAD<br>LENGTH | G<br>ACROMIAL<br>WIDTH | H<br>INFRA-<br>AXILLARY<br>WIDTH | I<br>NAVEL<br>HEIGHT | J<br>WAIST<br>HEIGHT | L<br>LENGTH<br>FROM BACK<br>TO ARM<br>CENTER | Q<br>RATIO OF LONG<br>AXIS AND SHORT<br>AXIS OF<br>ABDOMINAL<br>CIRCUMFERENCE |
|---|---|---|---|---|---|---|---|---|---|---|---|---|---|
| **MEN** 20S | 1.00 | Am2 | Bm2 | Cm2 | Dm2 | Em2 | Fm2 | Gm2 | Hm2 | Im2 | Jm2 | Lm2 | Qm2 |
| 30S | 1.00 | Am3 | Bm3 | Cm3 | Dm3 | Em3 | Fm3 | Gm3 | Hm3 | Im3 | Jm3 | Lm3 | Qm3 |
| 40S | 1.00 | Am4 | Bm4 | Cm4 | Dm4 | Em4 | Fm4 | Gm4 | Hm4 | Im4 | Jm4 | Lm4 | Qm4 |
| 50S | 1.00 | Am5 | Bm5 | Cm5 | Dm5 | Em5 | Fm5 | Gm5 | Hm5 | Im5 | Jm5 | Lm5 | Qm5 |
| 60S | 1.00 | Am6 | Bm6 | Cm6 | Dm6 | Em6 | Fm6 | Gm6 | Hm6 | Im6 | Jm6 | Lm6 | Qm6 |
| 70S | 1.00 | Am7 | Bm7 | Cm7 | Dm7 | Em7 | Fm7 | Gm7 | Hm7 | Im7 | Jm7 | Lm7 | Qm7 |

| | HEIGHT | A<br>UNDER<br>NECK | B<br>LEG | C<br>ARM | D<br>HEAD<br>WIDTH | E<br>HEAD<br>LENGTH | F<br>FORE-<br>AND-AFT<br>HEAD<br>LENGTH | G<br>ACROMIAL<br>WIDTH | H<br>INFRA-<br>AXILLARY<br>WIDTH | I<br>NAVEL<br>HEIGHT | J<br>WAIST<br>HEIGHT | L<br>LENGTH<br>FROM BACK<br>TO ARM<br>CENTER | Q<br>RATIO OF LONG<br>AXIS AND SHORT<br>AXIS OF<br>ABDOMINAL<br>CIRCUMFERENCE |
|---|---|---|---|---|---|---|---|---|---|---|---|---|---|
| **WOMEN** 20S | 1.00 | Af2 | Bf2 | Cf2 | Df2 | Ef2 | Ff2 | Gf2 | Hf2 | If2 | Jf2 | Lf2 | Qf2 |
| 30S | 1.00 | Af3 | Bf3 | Cf3 | Df3 | Ef3 | Ff3 | Gf3 | Hf3 | If3 | Jf3 | Lf3 | Qf3 |
| 40S | 1.00 | Af4 | Bf4 | Cf4 | Df4 | Ef4 | Ff4 | Gf4 | Hf4 | If4 | Jf4 | Lf4 | Qf4 |
| 50S | 1.00 | Af5 | Bf5 | Cf5 | Df5 | Ef5 | Ff5 | Gf5 | Hf5 | If5 | Jf5 | Lf5 | Qf5 |
| 60S | 1.00 | Af6 | Bf6 | Cf6 | Df6 | Ef6 | Ff6 | Gf6 | Hf6 | If6 | Jf6 | Lf6 | Qf6 |
| 70S | 1.00 | Af7 | Bf7 | Cf7 | Df7 | Ef7 | Ff7 | Gf7 | Hf7 | If7 | Jf7 | Lf7 | Qf7 |

# FIG.5

FIG.6B

HEIGHT

L

FIG.6A

INFRA-AXILLARY
THICKNESS

A
B
C
D
E
F
G
H
I
J
2x
2y

FIG.7

FIG.8A

FIG.8B

FIG.9A

FIG.9B

FIG.10B

FIG.10A

FIG.11

EP 2 687 158 B1

**FIG.12**

**FIG.13A**

**FIG.13B**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005261488 A
- US 20110295144 A1